# EUROPEAN PATENT APPLICATION

(11) **EP 4 736 741 A1**
(43) Date of publication of application: **06.05.2026**
(21) Application number: 24209436.5
(22) Date of filing: 29.10.2024
(51) Int. Cl.: A61B 5/00, A61B 5/1495, G01N 21/27

(54) **SPECTROSCOPY SYSTEM AND METHOD USING THE SAME**

(71) Applicant: Comind Technologies Limited, London EC1R 5EJ (GB)
(72) Inventor: ETARD, Octave, London, EC1R 5EJ (GB); AVTZI, Stella, London, EC1R 5EJ (GB)
(74) Representative: Mathys & Squire

(57) **Abstract**

A spectroscopy system comprising: a light delivery channel; a light receiving channel; and a reflective element; wherein the spectroscopy system is configurable in: a calibration arrangement in which light delivered from the light delivery channel is reflected from the reflective element and received in the light receiving channel; and a measurement arrangement in which light delivered from the light delivery channel travels through an object to be analysed and is received in the light receiving channel.

## Description

### Technical Field

The present disclosure relates to systems and methods for spectroscopy. In particular, the present disclosure relates to systems and methods relating to calibration and measurement of an optical spectroscopy system, such as a time-resolved spectroscopy system and/or an interferometric near infrared spectroscopy system (`iNIRS').

### Background

Spectroscopy methods such as iNIRS, diffuse correlation spectroscopy ('DCS'), time-resolved DCS ('TD-DCS') or time-resolved NIRS ('TD-NIRS') can be used to infer properties of an object by directing light from a light source, such as a laser, towards that object and then using a detector to measure corresponding properties of the light received from that object. The received light may include some of the light which was directed towards the object from the light source, and which subsequently scattered from the object and towards the detector. By monitoring this received light over time, and how the received light signals may change, one or more properties of the object can be inferred. For example, GB2619063 discloses non-invasive intracranial pressure sensing systems and methods which utilise iNIRS for non-invasively determining the intracranial pressure of a subject.

It is desirable to provide improvements to such spectroscopic systems and methods.

### Summary

Aspects of the disclosure are set out in the independent claims and optional features are set out in the dependent claims. Aspects of the disclosure may be provided in conjunction with each other, and features of one aspect may be applied to other aspects.

In an aspect, there is provided, a spectroscopy system comprising: a light delivery channel; a light receiving channel; and a reflective element. The spectroscopy system is configurable in: a calibration arrangement in which light delivered from the light delivery channel is reflected from the reflective element and received in the light receiving channel; and a measurement arrangement in which light delivered from the light delivery channel travels through an object to be analysed and is received in the light receiving channel.

Embodiments may enable the spectroscopy system to perform calibration using its own components. In particular, the spectroscopy system may itself have the ability to perform a calibration measurement using the same components and arrangement of components as would be used for analysing an object to be measured (except with the reflective element not impeding the delivery of light towards and from the object).

The system may comprise a controller configured to determine an indication of an instrument response function (`IRF') for the system based on light received in the light receiving channel when in the calibration arrangement. The controller may be configured to analyse the object based on both: (i) light signals received from the object in the light receiving channel, and (ii) the determined instrument response function. For example, the controller may be configured to determine sample data for the object, wherein that sample data comprises data obtained for the object which has been calibrated using the IRF data. For example, the controller may be configured to obtain sample time-of-flight data.

The system may comprise a probe arranged to couple (e.g. carry at least a portion of) one or both of the light delivery channel and the light receiving channel to a surface. For example, the probe may house a portion of both the light delivery channel and the light receiving channel. The reflective element may be removably couplable to the probe and/or a component arranged to couple the probe to a surface of the object. Removing the reflective film may permit light to travel from the light delivery channel, e.g. towards the object. The probe may be configured to be coupled to the reflective element in the calibration arrangement. The probe may be configured to be coupled to the object to be analysed in the measurement arrangement. Transitioning from the calibration arrangement to the measurement arrangement may comprise removing the reflective element from the probe. The reflective element may comprise a tab to facilitate removal of the reflective element from the probe. The reflective element may be arranged in a first position (for the calibration arrangement) in which it reflects light from the light delivery channel to the light receiving channel. The reflective element may be arranged in a second position (for the measurement arrangement) in which it does not impede the light path to/from the object to be analysed, e.g., in the second position the reflective element may have been removed from the probe. The probe may comprise a coupling surface configured to couple to: (i) the reflective element in the calibration arrangement, and (ii) the object to be analysed in the measurement arrangement. The coupling surface may comprise an adhesive.

The probe may comprise at least one intervening layer arranged between the light delivery channel and the reflective element and/or the object. That is, the intervening layer may be interposed between the light delivery channel and the reflective element (when operating in the calibration arrangement) and/or between the light delivery channel and the object (when operating in the measurement arrangement). Likewise, the at least one intervening layer may be interposed between the light receiving channel and the reflective element/object. The intervening layer may be optically diffusive or optically transparent. At least one of the reflective element and the intervening layer may comprise an optical diffuser. For example, the reflective element may comprise a diffusive reflector, e.g. in which case the intervening layer may be optically transparent or optically diffusive. For example, the reflective element may comprise a specular reflector, e.g. in which case the intervening layer may be optically diffusive.

The reflective element may comprise a reflective film. The spectroscopy system may be a time-resolved spectroscopy system, such as an iNIRS system. The system may be arranged to provide diffusion of the light between the light delivery channel and the light receiving channel. For this, the system may comprise a diffuser layer arranged to diffuse light output from the light delivery channel. The diffuser layer may comprise a mylar film. The diffuser layer may be located between the light delivery channel and the reflective film (or the object to be analysed when in the measurement arrangement). In which case, the reflective element may comprise a specular reflector. Additionally, or alternatively, the reflective element may comprise a diffuse reflector. In which case, the diffuser layer may optionally be omitted or the layer may instead be provided by a layer of optically transparent or guiding material.

In an aspect, there is provided a head mount for a human or animal head, the head mount comprising: a light delivery channel; a light receiving channel; and a reflective element. The head mount is configured to be coupled to a head of a human or animal for analysis of their brain, and wherein the head mount is configurable in: a calibration arrangement in which light delivered from the light delivery channel is reflected from the reflective element and received in the light receiving channel; and a measurement arrangement in which at least some light delivered from the light delivery channel travels through a portion of the brain of the human or animal head and is received in the light receiving channel. The head mount may comprise any portion of a spectroscopy system disclosed herein.

In an aspect, there is provided a method (e.g. a spectroscopy method) of using a spectroscopy system comprising: (i) a light delivery channel, (ii) a light receiving channel, and (iii) a reflective element, the method comprising: operating in a calibration arrangement in which light is delivered from the light delivery channel, reflected from the reflective element, and received in the light receiving channel; and operating in a measurement arrangement in which light is delivered from the light delivery channel, travels through an object to be analysed, and is received in the light receiving channel.

Light delivered from the light delivery channel may be diffused prior to being received in the light receiving channel (when in the calibration arrangement). For example, the system may comprise at least one component which provides diffusion of light, e.g. when operating in the calibration arrangement. For this, the light may be diffused by a diffuser layer and/or the reflective element may comprise a diffuser (arranged to provide diffuse reflection of light). The diffuser layer may be positioned between the light delivery channel and the reflective element. Additionally, or alternatively, the diffusion may be provided by a diffusive reflector. An optically transparent layer or optically guiding layer may be provided between the light delivery channel and the reflective element/object to be analysed. Light delivered from the light delivery channel may be diffused and/or guided prior to travelling through the object in the measurement arrangement. The light may be diffused and/or guided using a diffuser layer positioned between the light delivery channel and the object. The method may comprise transitioning from operating in the calibration arrangement to operating in the measurement arrangement. Said transitioning may comprise removing the reflective element. Removing the reflective element may comprise detaching the reflective element from a component which carries the light delivery channel. For example, the probe may include the light delivery channel, and said detaching may comprise detaching the reflective element from the probe. Additionally, or alternatively, another component may be included to couple the probe to the object, and the reflective element may be coupled to that component. In which case, removing the reflective element may comprise detaching the reflective element from said another component (e.g. before then using said component to couple the light delivery channel to the object). The method may comprise determining an indication of an instrument response function for the spectroscopy system based on light received in the light receiving channel when operating in the calibration arrangement. Analysing the object in the measurement arrangement may be based on both: (i) light signals received from the object in the light receiving channel, and (ii) the determined instrument response function.

Embodiments of the present disclosure may comprise a method for operating a spectroscopy system, the method comprising using the spectroscopy system in a calibration arrangement, comprising delivering light through a light delivery channel. Embodiments may also comprise diffusing the delivered light using either: (i) a diffuser layer positioned between the light delivery channel and a removable reflective element, and/or (ii) the reflective element wherein the reflective element comprises a diffusive reflector. Embodiments may also comprise reflecting the diffused light from the removable reflective element or diffusively reflecting light from the reflective element. Embodiments may also comprise receiving the reflected light in a light receiving channel. Embodiments may also comprise determining an indication of an instrument response function for the spectroscopy system based on the light received in the light receiving channel. Embodiments may also comprise using the spectroscopy system in a measurement arrangement, comprising detaching the removable reflective element to configure the spectroscopy system in the measurement arrangement. Embodiments may also comprise delivering light through the light delivery channel towards an object to be analysed. Embodiments may also comprise receiving light from the object in the light receiving channel for analysing. Embodiments may also comprise determining an indication of one or more properties of the object based on the light received in the light receiving channel. In some embodiments, the received light may comprise at least a portion of the light delivered towards the object after it has travelled through the object.

In some embodiments, the delivered light originates from a laser. In some embodiments, the delivered light may be in the near-infrared region. In some embodiments, the method may comprise sweeping a wavelength of the delivered light. In some embodiments, the diffuser layer may comprise mylar or another film operable to diffuse light, or the diffusive layer may be non-diffusive but configured to guide the delivered light from the light delivery channel to the light receiving channel. For example, the reflective element may comprise a diffusive reflector (e.g. configured to provide diffuse reflection without the diffuser layer). In some embodiments, the removable reflective element may comprise a reflective film that may be removably couplable to the probe. In some embodiments, the reflective film may comprise an aluminium coating, a metallic surface, a woven fiberglass surface finish, or another reflective coating. In some embodiments, the removable reflective element may comprise a tab operable for manual removal of the removable reflective element. In some embodiments, the object may be biological tissue, a human body, an animal body, or brain tissue. In some embodiments, the one or more properties of the object may comprise an absorption coefficient, a reduced scattering coefficient, a mean time-of-flight, total intensity, and/or intracranial pressure. In some embodiments, the determining an indication of one or more properties of the object may be further based on the determined indication of the instrument response function for the spectroscopy system. In some embodiments, the spectroscopy system may comprise an iNIRS system.

Embodiments of the present disclosure may also comprise a spectroscopy system, the system comprising a calibration arrangement, comprising a light delivery channel to deliver light. Embodiments may also comprise a diffuser layer positioned between the light delivery channel and a removable reflective element, the diffuser layer operable to diffuse the delivered light. In some embodiments, the removable reflective element may be operable to reflect the diffused light. Additionally, or alternatively, the reflective element may comprise a diffuse reflector arranged to provide diffuse reflection of light (e.g. without use of a diffuser layer). Embodiments may also comprise a light receiving channel operable to receive the reflected light. Embodiments may also comprise a controller configured to determine an indication of an instrument response function for the spectroscopy system based on the light received in the light receiving channel. Embodiments may also comprise a measurement arrangement, comprising detaching the removable reflective element to configure the spectroscopy system in the measurement arrangement. In some embodiments, the light delivery channel operable to deliver light towards an object to be analysed. In some embodiments, the light receiving channel for the analysing is operable to received light from the object. Embodiments may also comprise a controller configured to determine an indication of one or more properties of the object based on the light received in the light receiving channel. In some embodiments, the received light may comprise at least a portion of the light delivered towards the object after it has travelled through the object.

In some embodiments, the delivered light originates from a laser. In some embodiments, the delivered light may be in the near-infrared region. In some embodiments, the delivered light may comprise a wavelength swept emission. In some embodiments, the diffuser layer may comprise mylar or another film operable to diffuse light, or the diffusive layer may be non-diffusive but configured to guide the delivered light from said light delivery channel to the light receiving channel (and e.g. with a diffuse reflector). In some embodiments, the removable reflective element may comprise a reflective film that may be removably couplable to the probe. In some embodiments, the reflective film may comprise an aluminium coating, a metallic surface, a woven fiberglass surface finish, or another reflective coating. In some embodiments, the removable reflective element may comprise a tab operable for manual removal of the removable reflective element. In some embodiments, the object may be biological tissue, a human body, an animal body, or brain tissue. In some embodiments, the one or more properties of the object may comprise an absorption coefficient, a reduced scattering coefficient, a mean time-of-flight, total intensity, and/or intracranial pressure. In some embodiments, the determining an indication of one or more properties of the object may be further based on the determined indication of the instrument response function for the spectroscopy system. In some embodiments, the spectroscopy system may comprise an iNIRS system.

Aspects may also provide one or more computer program products comprising computer program instructions configured to program a spectroscopy system to perform any method disclosed herein.

### Figures

Some examples of the present disclosure will now be described, by way of example only, with reference to the figures, in which:
Fig. 1 is a schematic diagram of a portion of a spectroscopy system.
Fig. 2a is a schematic diagram of a spectroscopy system in a calibration arrangement.
Fig. 2b is a schematic diagram of a spectroscopy system in a measurement arrangement.
Fig. 3 shows three graphs illustrating example time of flight distributions.

In the drawings like reference numerals are used to indicate like elements.

### Specific Description

Embodiments are directed to spectroscopy systems, as well as methods of operating those spectroscopy systems, in which a reflective element is included as part of the spectroscopy system for selectively being used to perform a system calibration. For this calibration, light is delivered towards the reflective element of the spectroscopy system from a light delivery channel, and some of this light is reflected from the reflective element into a light receiving channel. In so doing, this light which is received in the light receiving channel has not been delivered to an object to be analysed. Instead, it has been delivered to a known reflective surface. As a result, the obtained data from these received light signals may be indicative of inherent properties of the optical system itself, such as an instrument response function (`IRF'), and so may be used for performing a system calibration. When using the system to obtain measurements of an object to be analysed, light is delivered from the light delivery channel into said object, where some of this light is reflected and/or scattered and then received within the light receiving channel. The received light may be processed to obtain an indication of one or more properties of the object. The data obtained during the calibration, such as the calculated IRF, may be used to further process data obtained from measurements of the object to be analysed (e.g., to account for the IRF).

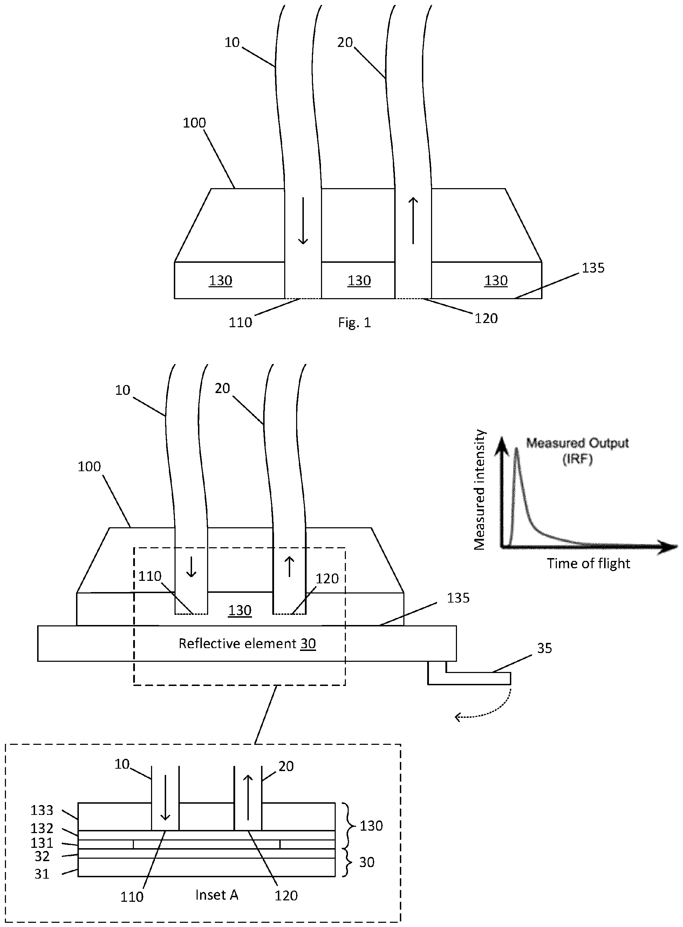

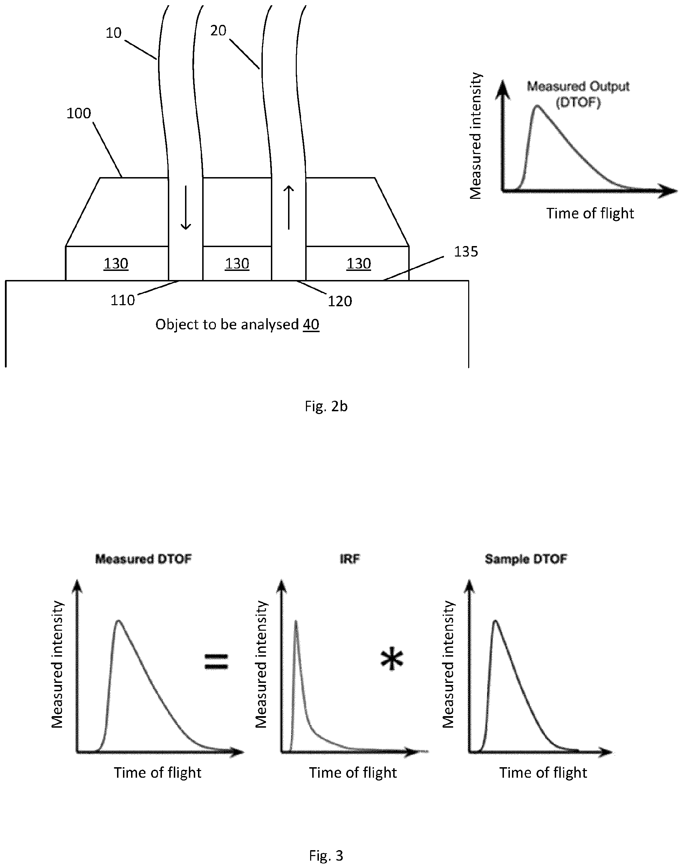

A portion of an example spectroscopy system will now be described with reference to Fig. 1. After, that spectroscopy system will be described for performing calibration with reference to Fig. 2a, and for performing measurement with reference to Fig. 2b.

The spectroscopy system includes a light delivery channel 10 and a light receiving channel 20. A light delivery channel end 110 is shown in Fig. 1, as is a light receiving channel end 120. The spectroscopy system also includes a probe 100. The probe 100 includes a coupling element 130 having a coupling surface 135.

The probe 100 houses a portion of the light delivery channel 10. The light delivery channel 10 may comprise an optical fibre. Although not shown in Fig. 1, the light delivery channel 10 is coupled to a light source, such as a laser. For example, at one end, the light delivery channel 10 is coupled to the light source, and the other end of the light delivery channel 10 is the light delivery channel end 110. The probe 100 holds the portion of the light delivery channel 10 at or near to the light delivery channel end 110. The light delivery channel end 110 is held by the probe 100.

The probe 100 houses a portion of the light receiving channel 20. The light receiving channel 20 may comprise an optical fibre. Although not shown in Fig. 1, the light receiving channel 20 is coupled to a detector, such as a photodetector. For example, at one end, the light receiving channel 20 is coupled to the detector, and the other end of the light receiving channel 20 is the light receiving channel end 120. The probe 100 holds the portion of the light receiving channel 20 at or near to the light receiving channel end 120. The light receiving channel end 120 is held by the probe 100.

The light receiving channel end 120 may be spaced apart from the light delivery channel end 110. For example, the light receiving channel end 120 may be more than a threshold distance away from the light delivery channel end 110. In other examples, the two channel ends 110, 120 may be located adjacent to each other (e.g., to obtain a 'null' measurement associated with zero channel separation).

The probe 100 comprises a proximal end and a distal end. The coupling element 130 is located at the proximal end of the probe 100. The coupling surface 135 is a proximal surface of the probe 100 (and also a proximal end of the coupling element 130 itself). In this sense, 'proximal' refers to being closer to the object to be illuminated (e.g., the reflective element 30 of Fig. 2a or the object 40 of Fig. 2b), with 'distal' being further away from said object. The probe 100 may comprise a body which extends distally away from the coupling element 130. The body of the probe 100 may house a portion of one or both light channels.

As will be described in more detail below in relation to Figs. 2a and 2b, the system may comprise a reflective element, and the probe 100 may be operated in either a calibration arrangement (which uses the reflective element) or a measurement arrangement (which uses an object to be analysed). In both arrangements, a proximal end of probe 100 may be coupled to a surface that is to be illuminated (that surface being either a surface of the reflective element or a surface of the object to be analysed). For example, in either arrangement, the coupling surface 135 may couple the probe 100 to said surface that is to be illuminated, e.g., the coupling surface 135 may contact that surface. The light delivery channel end 110 and/or the light receiving channel end 120 may be coupled to, e.g., directly contact, that surface. Alternatively, the light delivery channel end 110 and/or the light receiving channel end 120 may be coupled to that surface via an intermediary component or layer, such as a diffuser layer.

The coupling element 130 may extend proximally beyond the light delivery channel end 110 and/or the light receiving channel end 120 or the light delivery channel end 110 and/or the light receiving channel end 120 may be flush with the coupling element 130 (or even extend beyond the proximal end of the coupling element 130). In Fig. 1, the light delivery channel end 110 and the light receiving channel end 120 are flush with the coupling surface 135 of the coupling element. The channels 10, 20 may extend through an opening in the coupling element 130 (e.g., a region in which there is no material of the coupling element 130). The coupling element 130 may be located in the regions around (e.g., which at least partially laterally surround) the channels 10, 20. For example, a region between the two channels 10, 20 may comprise coupling element 130 material, as may regions on either side of the channels 10, 20. Alternatively, and as described in more detail below in relation to Fig. 2a, the channel ends 110, 120 may be distal of the coupling surface 135.

The spectroscopy system may be a time-resolved spectroscopy system, e.g., for diffuse optical spectroscopy. For example, the spectroscopy system may be for time domain near infrared spectroscopy ('TD-NIRS'), time domain diffuse correlation spectroscopy ('TD-DCS'), time domain diffuse optical tomography (`TD-DOT'), or interferometric near infrared spectroscopy ('iNIRS'). In particular, the spectroscopy system may be an iNIRS system.

The light delivery channel 10 and the light receiving channel 20 may each comprise an optical fibre. The light delivery channel 10 may comprise a single-mode fibre or a multi-mode fibre. The light receiving channel 20 may comprise a single-mode fibre or a multi-mode fibre. The spectroscopy system may utilise near infrared light. For example, the spectroscopy system may comprise a light source, e.g., a laser, operable to output light in a near-infrared region. The light delivery channel end 110 is spaced apart from the light receiving channel end 120. The two may be spaced apart by a distance (e.g., a lateral offset) selected based on a desired penetration depth for light into an object to be analysed. For example, for deeper penetration, the two may be spaced further apart.

The probe 100 may be a multi-component probe 100. For example, the probe 100 may hold both the light delivery channel 10 and the light receiving channel 20. The probe 100 also comprises the coupling element 130 (and coupling surface 135) for connecting (e.g., adhering) the probe 100 to other objects.

The coupling element 130 may comprise an adhesive. Although not shown in Fig. 1, the coupling element 130 may comprise a plurality of layers. One of the layers may comprise an adhesive layer. For example, the most proximal layer (i.e., the layer which will be closest to the object to be analysed) may comprise an adhesive. The coupling surface 135 may comprise an adhesive, e.g., it may be an adhesive layer. The adhesive may comprise a double-sided adhesive tape, such as double-sided tape sold under the trademark of 3M. The adhesive may comprise a photo-stable adhesive.

The coupling element 130 may be flexible. For example, the coupling element 130 may comprise a layer of a flexible material, such as a foam. The coupling element 130 may comprise a flexible (e.g., foam) layer and an adhesive layer, e.g., with the adhesive layer being located proximally of the flexible (e.g., foam) layer. The coupling surface 135 (e.g., the adhesive layer) may be approximately planar (e.g., flat). The coupling element 130 may be arranged to permit distortion, such as bending and/or compression, of the coupling surface 135, e.g., due to the flexibility of the coupling element 130. The coupling element 130 may not utilise an adhesive for the coupling surface 135, e.g., where no adhesion is needed between the probe 100 and the surface to be illuminated.

In Fig. 1, the coupling element 130 (and thus coupling surface 135) is shown as being offset from the light delivery channel end 110 and the light receiving channel end 120. That is, the coupling element 130 is shown as being laterally offset from both light channel ends. As such, there is an opening in the coupling element 130 through which the light delivery channel 10 extends, e.g., towards an object to which the probe 100 is coupled. Likewise, the light receiving channel 20 may extend through an opening in the coupling element 130. For example, there may be one opening through which both channels 10, 20 extend or there may be two separate openings (e.g., one for each channel 10, 20).

In Fig. 1, the channels 10, 20 are each shown as extending through an opening in the coupling element 130, but it is to be appreciated in the context of the present disclosure that this may not be the case. For example, the channel ends 110, 120 may be located distally of the coupling surface 135, and there may be a length of the coupling element 130 through which light emitted from the channel ends 110, 120 must travel. In which case, the coupling element 130 may comprise no material in this region or it may comprise a material which is at least partially optically transparent, e.g., so as to still permit light to travel between the light channels and the surface to be illuminated.

For example, the coupling element 130 may comprise an optical diffuser. For this, a layer of the coupling element 130 may comprise an optical diffuser layer. The light channel ends 110, 120 may be coupled to (e.g., in direct contact with) the optical diffuser (e.g., with that optical diffuser intervening between the channel ends 110, 120 and the surface to be illuminated). For example, the optical diffuser may extend across the majority, or the entire spatial extent, of the cross-sectional area encompassing the channel ends 110, 120 between those ends 110, 120 and the coupling surface 135. The optical diffuser may comprise at least one layer of an optically diffusive material, such as Mylar (TM). For example, the optical diffuser may comprise a film of optically diffusing material.

As will be described in more detail below, the provision of an optical diffuser may provide diffusion of light emitted from the light delivery channel 10, thereby to permit the use of a specular reflector. Additionally, or alternatively, the reflector itself may comprise a diffuse reflector.

The spectroscopy system is configured to direct light from a light source through the light delivery channel 10 towards an object to be analysed. At least some of that light will be reflected and/or scattered and received within the light receiving channel 20 where that received light will be provided to a detector. The spectroscopy system is configured to determine a time-of-flight distribution for light received at the detector. Each time-of-flight distribution may provide a distribution of flight times for a plurality of different incident photons at the detector. For example, a plurality of photons may be emitted through the light delivery channel 10, and some of these photons may take different scattering paths between leaving the light delivery channel 10 and entering the light receiving channel 20. The time-of-flight may vary for each individual photon path (e.g., due to different numbers of scattering events etc.), and this may be evident from the obtained distribution.

For an iNIRS system, the light source provides wavelength swept emission of coherent light.

Some of this light is provided along a reference channel towards, while the remainder of the light is delivered through the light delivery channel 10 (e.g., towards an object to be analysed). Some of the light delivered to the object will be received within the light receiving channel 20 as sample light, and then subsequently combined with reference light from the light source. Due to the wavelength sweeping of the light source and the difference in sample and reference arm lengths, the wavelength of the sample light will differ from the wavelength of the reference light. The magnitude of this difference in wavelength will correspond to the difference in length for the two path lengths, i.e., to the particular path the sample light has taken. This difference will be apparent in resulting beat frequencies in the combined light signal. By obtaining an indication of the different beat frequencies received, the detector may obtain a time-of-flight distribution.

The light delivery channel 10 is arranged to deliver light towards an object to be analysed. For this, the light delivery channel 10 may be arranged to deliver light through the coupling element 130 (e.g., through the light delivery channel 10 in an opening in the coupling element 130 or from the light delivery channel 10 and at least partially through the coupling element 130) towards an object to which the coupling surface 135 is coupled.

The light receiving channel 20 is arranged to receive light from an object to be analysed. For example, the light receiving channel 20 may be arranged to receive light from the object which was delivered to the object through the light delivery channel 10. For this, the light delivery channel 10 may be arranged to receive light through the coupling element 130 (e.g., through the light receiving channel 20 in an opening in the coupling element 130 or at least partially through the coupling element 130 and then into the light receiving channel 20) from an object to which the coupling surface 135 is coupled.

The probe 100 is arranged to couple both the light delivery channel 10 and the light receiving channel 20 to a surface of an object to be analysed. The probe 100 may be configured to hold the channels in a fixed spatial arrangement with respect to the object. For example, the probe 100 may be configured to adhere to the surface of the object to be analysed. The probe 100 may be configured to either: (i) couple the channel ends 110, 120 directly to said surface, e.g., so that they are in direct contact with said surface, or (ii) couple the channel ends 110, 120 to said surface via one or more intermediary layers. Such intermediary layer(s) may comprise a diffuser layer (e.g. to diffuse the light emitted from the light delivery channel 10) and/or an optically transparent and/or guiding layer (e.g. to guide the light emitted from the light delivery channel 10).

For this, the coupling element 130 is configured to couple to a surface of an object. The coupling element 130 is configured to attach itself and the probe 100 to the surface of said object. The coupling surface 135 is arranged to provide a surface which will contact, e.g., and adhere to, the surface of the object to be analysed. For example, the coupling surface 135 may comprise an adhesive configured to adhere to the surface of the object to be analysed. The coupling surface 135 may comprise an adhesive layer (e.g., a layer which contains at least some adhesive material) arranged to adhere the coupling element 130 (as well as the probe 100 itself) to the surface of the object to be analysed. In other words, the coupling surface 135 is configured to provide a surface which may be placed on other objects, e.g., and where the coupling surface 135 is configured to adhere the probe 100 to the object onto which the coupling surface 135 has been placed.

The coupling element 130 may be configured to facilitate adhesion to an uneven surface. For example, the coupling element 130 may be configured to conform to a surface of the object to which it is to be coupled. For this, the coupling element 130 may comprise one or more layers of material located distally of the coupling surface 135 (e.g., located further away from the object to be analysed than the coupling surface 135). For example, the probe 100 may comprise one or more layers of material between the coupling surface 135 and a main body of the probe 100. The layer(s) of material may comprise a layer of a flexible and/or compressible material, e.g., a foam. The coupling element 130 (and the flexible material therewithin) may be configured to permit localised distortion and/or bending thereby to enable the coupling surface 135 to conform to the surface of the object to which it is to be coupled.

As described above, the spectroscopy system includes a probe 100 which is configured to be coupled to a surface of an object to be analysed. For this, the coupling surface 135 of the probe 100 is configured to adhere to the surface of the object to be analysed thereby to hold the probe 100 in a fixed spatial orientation with respect to the object to be analysed. The spectroscopy system may be configured to direct light towards said object (through the light delivery channel 10), and to receive some of this light which has scattered from the object (through the light receiving channel 20). The spectroscopy system may be configured to determine one or more time-of-flight distributions for these received light signals.

As will now be described in more detail below in relation to Fig. 2a, the spectroscopy system may comprise a reflective element 30 to be used for a calibration process. After this, and with reference to Figs. 2b and 3, a measurement process using the spectroscopy system (and its calibration) will be described.

Fig. 2a shows the portion of the spectroscopy system shown in Fig. 1. For the sake of brevity, the same components will not be described again.

In addition, the spectroscopy system of Fig. 2a also includes a reflective element 30. The reflective element 30 may comprise a tab 35.

Also, as shown in Inset A of Fig. 2a, the reflective element 30 may comprise a reflective layer 31 and a first diffuser layer 32. Likewise, the coupling element 130 may comprise an adhesive layer 131, a second diffuser layer 132, and a flexible layer 133. In the example of Fig. 2a, one or more diffuser layers 32, 132 may be included, but as will be described below, one or more of these layers 32, 132 may be replaced, e.g. with an optically transparent and/or guiding layer, and the reflective element 30 may comprise a diffuse reflector.

The reflective element 30 may comprise a multi-layered structure. As shown in Fig. 2a, this structure may comprise a reflective layer 31 and a first diffuser layer 32. Although not shown, the reflective element 30 may also comprise one or more regions of adhesive or adhesive layers for connecting adjacent layers of the structure to each other. For example, adhesive (e.g., a layer) may be included between the reflective layer 31 and the first diffuser layer 32 (e.g., to adhere the two layers to each other). Likewise, adhesive (e.g., a layer) may be included on a distal side of the first diffuser layer 32 (e.g., on the coupling element 130 facing side of the reflective element 30). The first diffuser layer 32 is located distally (e.g., on a probe 100-facing side) of the reflective layer 31. In other words, the first diffuser layer 32 is located between the light channels and the reflective layer 31 (when the reflective element 30 is coupled to the probe 100).

The reflective layer 31 may comprise a film. For example, the reflective layer 31 of the reflective element 30 may be provided by a film of optically reflective material. The reflective layer 31 of the reflective element 30 may comprise a metallic surface. For example, the reflective element 30 may comprise an aluminium coating. The reflective layer 31 of the reflective element 30 may have a woven fiberglass surface finish. For example, the reflective layer 31 of the reflective element 30 may be formed of a glass fiber fabric with a reflective coating, such as an aluminium coating.

The reflective element 30 may include an optional first diffuser layer 32. The first diffuser layer 32 may comprise an optically diffusing material. The first diffuser layer 32 may comprise a film. For example, the first diffuser layer 32 may comprise a film of an optically diffusing material, such as Mylar (TM). The first diffuser layer 32 is located on a probe-facing side of the reflective layer 31. Additionally, or alternatively, the reflective element 30 may comprise a diffuse reflector (e.g. arranged to provide diffuse reflection, such as without any additional diffuser layers).

The reflective element 30 may comprise a tab 35. The tab 35 may comprise a piece of material extending away from a main body of the reflective element 30. The tab 35 may comprise a piece of material not adhered to the coupling element 130 (e.g., via the coupling surface 135/adhesive layer 131). The tab 35 may comprise a flange of material. For example, the tab 35 may be connected to the reflective layer 31 (e.g., on a proximal side of the reflective layer 31 - i.e., the side further away from the probe 100). Additionally, or alternatively, the tab 35 may extend laterally away from one side of the reflective element 30.

The coupling element 130 may comprise a multi-layered structure. As shown in Fig. 2a, this structure may comprise an adhesive layer 131, a second diffuser layer 132 and a flexible layer 133. Although not shown, the coupling element 130 may also comprise one or more regions of adhesive or adhesive layers (in addition to adhesive layer 131) for connecting different layers of the structure to each other. For example, adhesive (e.g., a layer) may be included between the second diffuser layer 132 and the flexible layer 133 (e.g., to adhere the two layers to each other). Likewise, adhesive (e.g., a layer) may be included between the flexible layer 133 and a main body of the probe 100 (e.g., on a far side of the flexible layer 133 to the reflective element 30). One or more of the layers may comprise a liner. For example, the liner may comprise an additional piece of material at least partially surrounding said layer. For example, the flexible layer 133 may comprise a flexible element, such as a foam, which is at least partially wrapped in a liner.

The second diffuser layer 132 is located distally of the adhesive layer 131 (e.g., on a far side of the adhesive layer 131 to the reflective element 30). In other words, the second diffuser layer 132 is located between the light channels and the adhesive layer 131 (and thus also the reflective layer 31 when the reflective element 30 is coupled to the probe 100). The second diffuser layer 132 may be provided in addition to, or as an alternative to, the first diffuser layer 32. The second diffuser layer 132 may be provided a similar (e.g., the same) arrangement as the first diffuser layer 32 described above. As described in more detail below, both diffuser layers 32, 132 may be provided together or only one diffuser layer may be provided.

The flexible layer 133 may be located proximally or distally (distally in Fig. 2a) of the second diffuser layer 132. The flexible layer 133 is located distally of the adhesive layer 131. In other words, the flexible layer 133 is located between a main body of the probe 100 and the adhesive layer 131. The flexible layer 133 may comprise a flexible and/or compressible material, e.g., which may deform. For example, the flexible layer 133 may comprise a foam material. The flexible layer 133 may be located between the adhesive layer 131 and the ends of the light channels and/or the flexible layer 133 may surround the ends of the light channels in the same region (e.g., in the same layer). For example, as shown in Fig. 2a, the light channels 10, 20 may at least partially extend through the flexible layer 133 at their proximal ends. As such, the light channel ends 110, 120 may be located within the flexible layer 133 or flush with a proximal end surface of the flexible layer 133 (as shown in Fig. 2a). The light channel ends 110, 120 may directly contact the second diffuser layer 132.

The adhesive layer 131 may provide the coupling surface 135 of the coupling element 130. The adhesive layer 131 may comprise an adhesive, such as double-sided adhesive tape.

As shown in Inset A of Fig. 2a, the light delivery channel end 110 and the light receiving channel end 120 may be located within the flexible layer 133. The flexible layer 133 may extend around a periphery of the coupling element 130, e.g., to surround the light channels 10, 20. For example, the flexible material may extend around the circumference of each light channel 10, 20, with the light channels 10, 20 extending through a region in which there is no flexible material.

The second diffuser layer 132 may run at least partially perpendicular to the light channels 10, 20. The second diffuser layer 132 may be located between the light channel ends 110, 120 and the surface of the object coupled to the probe 100 (the reflective element 30 in Fig. 2a). The second diffuser layer 132 may be located on an optical path for light which has been emitted from the light delivery channel 10 towards the surface of the object coupled to the probe and/or on an optical path for light returning from the object towards the light receiving channel 20. The flexible layer 133 and the adhesive layer 131 may not be located in this region, e.g., they may not intervene between the light channel ends and the surface coupled to the probe 100. The adhesive layer 131 may also only span across some or all of the region surrounding the light channel ends 110, 120 (but not across the light channel ends 110, 120 themselves).

For the reflective element 30, the first diffuser and/or reflective layer 31 may span across a majority of the area of the reflective element 30.

The reflective element 30, once coupled to the coupling element 130 of the probe 100, will be located proximally of the probe 100. The adhesive layer 131 (/coupling surface 135) of the coupling element 130 is coupled to the reflective element 30. For example, the adhesive layer 131 may couple directly to the first diffuser layer 32 and/or there may be one or more intervening layers. When coupled to the coupling element 130 of the probe 100, the reflective layer 31 is aligned with the light channel ends 110, 120. That is, the reflective layer 31 spans across the area onto which light from the light delivery channel 10 is directed, e.g., so that the light delivery channel is directing light towards an area containing the reflective layer 31. Likewise, if included, the first diffuser layer 32 spans across this area, e.g., so that light delivered from the light delivery channel is directed towards an area containing a diffuser material (e.g., of the first diffuser layer 32). The same region may also comprise the reflective layer 31 (located proximally of the first diffuser layer 32).

The spectroscopy system is arranged to permit coupling between the light delivery channel 10, the light receiving channel 20 and the reflective element 30. In other words, the spectroscopy system is configured to enable an arrangement to be provided in which the reflective element 30 is coupled to the light delivery channel 10 and the light receiving channel 20. When coupled in this way, the reflective element 30 may be held in a fixed spatial arrangement with respect to the light delivery channel 10 and the light receiving channel 20. In this fixed arrangement, the reflective element 30 is configured to receive light from the light delivery channel 10 and to reflect this light towards the light receiving channel 20.

With the light channels coupled to the reflective element 30 (e.g., in the manner mentioned above) the system will be referred to as being in a 'calibration arrangement'.

When in the calibration arrangement, the spectroscopy system is configured to direct light (from the light source) towards the reflective element 30. That is, the spectroscopy system is configured to deliver light through the light delivery channel 10 and towards the reflective element 30. The reflective element 30 is configured to reflect at least some incident light from the light delivery channel 10. The spectroscopy system is arranged so that at least some of the reflected light will be received within the light receiving channel 20 (and provided to the detector). In other words, the spectroscopy system is configured to provide an optical path from the light source (through the light delivery channel 10) to the detector (through the light receiving channel 20) via the reflective element 30.

The coupling between the light delivery channel 10, the light receiving channel 20 and the reflective element 30 may thus provide a light path from source to detector which does not pass through an object to be analysed. Instead, the light delivered from the light delivery channel 10 is reflected from the reflective element 30 and received in the light receiving channel 20.

The spectroscopy system may be configured to operate in this calibration arrangement to obtain data for performing a system calibration. That is, the spectroscopy system may be configured to detect and process light signals received at the detector which have arrived via a reflection from the reflective element 30. For example, the spectroscopy system may be configured to obtain one or more time-of-flight distributions for these obtained light signals. An example distribution for this is shown in Fig. 2a (on the right-hand side of the figure). As shown, this distribution may link a measured intensity for received light to a time-of-flight taken for that light to reach the detector. This obtained distribution may be independent of any travel of photons through an object to be analysed, as instead the photons will have reflected from the reflective element 30.

The spectroscopy system may be configured to utilise time-of-flight data obtained when in the calibration arrangement for calibrating the spectroscopy system. In particular, the spectroscopy system may be configured to determine an indication of an instrument response function (`IRF') for the spectroscopy system based on this obtained data. As will be appreciated in the context of the present disclosure, the IRF may be dependent upon optical components used in the system, as well as control parameters for the light source (e.g., laser). For example, the contribution from optical components may arise due to photons travelling from the light source (e.g., laser) through the various optical components. For example, the contribution from the light source may depend upon a sweeping rate (repetition rate) of the sweeps performed by the light source and/or sweeping range (bandwidth that the light source sweeps) may influence the IRF.

The spectroscopy system may be configured to utilise any obtained calibration data (e.g., an obtained indication of an IRF for the system) for measurements obtained from a sample to be analysed. In other words, the system may be configured to utilise the reflective element 30 to obtain calibration data, e.g., by operating the light source and detector with the reflective element 30 arranged to provide a reflective path between the light delivery channel 10 and the light receiving channel 20.

In this sense, the spectroscopy system may comprise intrinsic calibration (e.g., IRF) measurement capabilities. For instance, through the inclusion of the reflective element 30 within the spectroscopy system, the spectroscopy system may be operated as normal (e.g., as if it were being used to obtain measurements from an object to be analysed), and the reflective element 30 may provide optical reflection, thereby to enable calibration data (e.g., an IRF) to be obtained for the system. The arrangement (spatial distribution) of the light delivery channel 10 and light receiving channel 20 need not be changed after calibration.

The probe 100 (the coupling element 130) and the reflective element 30 are configured to interact with each other to facilitate connection therebetween to configure the system in its calibration arrangement. That is, the probe 100 and reflective element 30 are connectable to each other to provide an optical path from the light delivery channel 10 to the light receiving channel 20 via the reflective element 30.

The spectroscopy system is configured to provide a removable connection between the reflective element 30 and the probe 100. That is, the reflective element 30 is removable from the probe 100 (e.g., so that the two are no longer connected). For example, the spectroscopy system is configured to enable the reflective element 30 to be coupled to the probe 100 to provide the calibration arrangement, and for the reflective element 30 to then be removed from the probe 100. The probe 100 is configured to be coupled to an object to be analysed after removal of the reflective element 30 (e.g., the removal of the reflective element 30 from the probe 100 is such that the probe 100 may still couple to another object). In other words, the reflective element 30 is removably couplable to the probe 100.

The tab 35 is configured to facilitate removal of the reflective element 30 from the probe 100. For example, the tab 35 is arranged to provide increased leverage for removing the reflective element 30 from the probe 100. The reflective element 30 may be configured to be peeled off from the probe 100. Removal of the reflective element 30 from the probe 100 may enable the probe 100 to be coupled to another object, such as an object to be analysed.

With the reflective element 30 not coupled to the probe 100, and the probe 100 coupled to a surface of an object to be analysed, the spectroscopy system will be referred to as being in a 'measurement arrangement'.

The spectroscopy system is configured for the reflective element 30 to be removed from the probe 100 to transition from the calibration arrangement towards the measurement arrangement. The probe 100 is configured to be coupled to a surface of an object to be measured when the reflective element 30 is not coupled thereto (e.g., once the reflective element 30 has been removed). In other words, the probe 100 is configured to be coupled to one of the reflective element 30 or the object to be analysed, and the probe 100 is configured to switch between which one of these two to which it is coupled. The tab 35 is configured to facilitate removal of the reflective element 30 from the probe 100 to assist with the transition of the spectroscopy system from the calibration arrangement to the measurement arrangement.

As shown in Inset A of Fig. 2a, each of the coupling element 130 and the reflective element 30 may be provided by multi-layered structures. The multi-layered structure of the reflective element 30 is designed to further facilitate operation of the system in the calibration arrangement. The multi-layered structure of the coupling element 130 may further facilitate operation in both the calibration and measurement arrangements.

The reflective layer 31 of the reflective element 30 is configured to provide optical reflection. The reflective layer 31 of the reflective element 30 is the surface/body from which light delivered by the light delivery channel 10 will reflect (e.g., so that it may return to the light receiving channel 20). The reflective layer 31 may be arranged so that, when the reflective element 30 is coupled to the probe 100, the reflective layer 31 provides optical reflection to reflect light back towards the light channels (e.g., so that light may travel from the light delivery channel 10 to the light receiving channel 20 via the reflective layer 31).

The first diffuser layer 32 is configured to optically diffuse light. For example, light which is incident on the first diffuser layer 32 may be diffused (scattered) into a plurality of different directions through its interaction with the first diffuser layer 32. The first diffuser layer 32 is located between the optical channels and the reflective layer 31. The first diffuser layer 32 is configured to diffuse light which has been delivered towards the reflective layer 31 from the light delivery channel 10. The first diffuser layer 32 is configured to diffuse light which has been reflected from the reflective layer 31 towards the light channels. The first diffuser layer 32 may be arranged so that at least some light which is incident on the first diffuser from the light delivery channel 10 is diffused on an optical path towards the light receiving channel 20. For example, the light delivery channel 10 and the light receiving channel 20 may be parallel, and they are spaced apart, and so the first diffuser layer 32 may be configured to facilitate the provision of an optical path from the light delivery channel 10 to the light receiving channel 20 via the reflective layer 31. For example, the first diffuser layer 32 may be arranged to avoid the need for a specular reflection path from the light delivery channel 10 to the light receiving channel 20. Instead, the first diffuser layer 32 is configured to provide scattering of light to facilitate this optical path.

The coupling element 130 of the probe 100 is configured to facilitate connection of the probe 100 to the reflective element 30 (when in the calibration arrangement) and the object to be analysed (when in the measurement arrangement). The coupling element 130 is configured to provide a removable connection. For this, the coupling element 130 is configured to couple, e.g., adhere, to a surface of the relevant body to which it is coupled. The probe 100 may be manually removed from said surface. The coupling element 130 is configured for multiple couplings, e.g., multiple adhesions. That is, the coupling element 130 is configured to be first coupled to one surface, before being removed from that surface and coupled to another surface. For instance, the coupling element 130 may be configured to first be coupled (adhered) to the reflective element 30 (for the calibration arrangement) before then being coupled (adhered) to the object to be analysed (for the measurement arrangement).

The adhesive layer 131 is configured to adhere to a surface it contacts. For example, the adhesive layer 131 may comprise one or more (e.g., a plurality of) regions of adhesive which will stick to a surface which contacts them. The adhesive layer 131 is securely attached to the rest of the coupling element 130 to permit removal of a surface coupled to the adhesive layer 131 without removing the adhesive layer 131 from the coupling element 130 as well. The adhesive layer 131 may be re-usable, e.g., so that it may be adhered twice or more times to a surface. Although not shown in Fig. 2a, the coupling element 130 may comprise one or more additional layers, such as additional non-adhesive layers and/or additional adhesive layers. For example, there may be additional intervening layers within the multi-layered structure of the coupling element 130, e.g., to provide further compressibility and/or flexibility, and/or provide structural support to other layers of the structure.

The second diffuser layer 132 may be configured to provide similar functionality to the first diffuser layer 32. In so doing, the second diffuser layer 132 is configured to optically diffuse light. The second diffuser layer 132 is located between the optical channels and the reflective layer 31 of the reflective element 30 (in the calibration arrangement) or between the optical channels and a surface of the object to be analysed (in the measurement arrangement). The second diffuser layer 132 is configured to diffuse light which is passing through it when travelling away from and/or towards the optical channels. The second diffuser layer 132 may be arranged to avoid the need for a specular reflection path from the light delivery channel 10 to the light receiving channel 20 (e.g., when operating in the calibration arrangement). Only one of the two diffuser layers may be provided. In which case, that one diffuser layer may be configured to provide optical diffusion mentioned above.

The flexible layer 133 may be configured to permit localised distortions in its material. For example, the flexible layer 133 may be flexible, compressible and/or bendable. The flexible layer 133 is arranged to conform to one or more uneven aspects of a surface which the probe 100 is to contact. For example, the flexible layer 133 is configured to absorb the differences in the uneven surface while enabling the probe 100 to retain its intended arrangement. The inclusion of the flexible layer 133 may be of particular use for the measurement arrangement, e.g., when the object to be analysed may have an uneven surface (whereas the reflective element 30 may be substantially planar and flat).

As described above, the spectroscopy system is configurable in a calibration arrangement (with the probe 100 coupled to the reflective element 30) and a measurement arrangement (with the probe 100 coupled to an object to be analysed).

An example of the system in the measurement arrangement will now be described with reference to Fig. 2b.

Fig. 2b shows the spectroscopy system coupled to an object 40 to be analysed. As compared to Fig. 2a, the reflective element 30 is not there, as the probe 100 is coupled to the object 40 instead. As with Fig. 2a, the coupling element 130 provides the connection to the surface of the body to which the probe 100 is coupled. In other words, the coupling element 130 is arranged to connect the probe 100 to the object 40 to be analysed. In Fig. 2b, the optical channel ends 110, 120 are shown as directly contacting the object to be analysed 40 (rather than one or more intervening layers, such as a second diffuser layer 132, as in Fig. 2a).

Although not shown in Fig. 2b, the coupling element 130 may include the same multi-layered structure shown in Fig. 2a. In which case, the adhesive layer 131 may be adhered to the surface of the object 40. The flexible layer 133 may be in a relaxed state, or there may be at least some localised distortion of this layer to accommodate any unevenness to the surface of the object 40. The second diffuser layer 132 may be arranged between the optical channels and the surface of the object 40 to be analysed.

As such, the same probe 100 may be coupled to the object 40 or the reflective element 30 in a similar manner. That is, the same adhesive may adhere the probe 100 to the surface of either the object 40 to be analysed or the reflective element 30. For example, the coupling element 130 (and coupling surface 135/adhesive layer 131) may be sequentially attachable to the reflective element 30 and the object 40. It will be appreciated that this may be performed in either order, but typically, the probe 100 will first be coupled to the reflective element 30 (calibration) before being coupled to the object 40 (measurement).

As already mentioned, the spectroscopy system is configured to deliver light from the light source through the light delivery channel 10 and to direct light received in the light receiving channel 20 to the detector. When using the spectroscopy system to analyse the object 40 (rather than reflective element 30), a greater proportion of the photons will penetrate beyond the surface of the object 40 and further inside. Photons which are ultimately received back from the object 40 may therefore be at least partially informative about an internal volume of the object 40.

For example, for highly scattering media, such as biological tissue, light will not travel in a straight line between the light delivery channel 10 and the light receiving channel 20, but rather this will follow a random walk from scattering event to scattering event. As a result, some photons travel a shorter path, and thus take less time to reach the light receiving channel 20, while others take a longer path, typically travelling deeper into the object 40 and taking longer to reach the light receiving channel 20. Again, the system may be configured to obtain a time-of-flight distribution containing a histogram of photon flight times. An example for this distribution is shown on the right-hand side of Fig. 2b. As compared to the distribution of Fig. 2a, the distribution in Fig. 2b is broader and has a longer tail. This shows that there are a greater range of flight times, as well as more longer flight times compared to the Fig. 2a arrangement.

An example operation of the spectroscopy system will now be described with reference to Figs. 2a, 2b and 3.

The spectroscopy system is first operated in a calibration arrangement. For this, the probe 100 is coupled to the reflective element 30, as shown in Fig. 2a (e.g., with the adhesive layer 131/coupling surface 135 of the coupling element 130 of the probe 100 adhered to the reflective element 30). A time-of-flight distribution is then obtained by delivering light through the light delivery channel 10 towards the reflective element 30 and detecting properties of the resulting light signals received through the light receiving channel 20. The resulting distribution is shown in Fig. 2a on the right-hand side, and as the middle distribution in Fig. 3. This distribution is used to provide an indication of the IRF for the spectroscopy system. The earliest non-zero value on this curve represents the minimum flight time through the system, and the width of this curve is related to the minimum precision to which the system can determine changes in time-of-flight (e.g., time-of-flight temporal resolution).

With this IRF data obtained from operating in the calibration arrangement, the system may then analyse other objects and use this IRF data to calibrate the measurements.

The reflective element 30 is then removed from the probe 100. For this, the reflective element 30 may be manually removed, e.g., peeled off, from the probe 100. For example, the tab 35 may be used to facilitate this removal of the reflective element 30. With the reflective element 30 removed, the coupling element 130 of the probe 100 will remain intact, and the adhesive layer 131/coupling surface 135 may be re-used for adhering the probe 100 to another surface. The probe 100 is then coupled to a surface of the object 40, as shown in Fig. 2b. One or more time-of-flight distributions may be obtained for the object 40. An example of one of these distributions is shown on the right-hand side in Fig. 2b, and as the left distribution in Fig. 3.

As can be seen from Fig. 3, the obtained time-of-flight distribution for the object 40 can be considered to be formed from a combination of two separate components: (i) the IRF (the middle curve), and (ii) a sample time-of-flight distribution associated with the absorption and scattering from the object 40 (the right curve). For example, the obtained time-of-flight distribution may comprise a convolution of the IRF distribution and the sample distribution. It is the sample distribution that is of most interest, since this may represent properties of the internal volume of the object 40 to be analysed.

The method may comprise processing the obtained distributions to identify the sample distribution. For example, the sample distribution may be extracted from the obtained distribution for the object 40 using the obtained IRF distribution. This may comprise a deconvolution (e.g., to deconvolve the IRF from the obtained distribution for the object 40), or another approach for accounting for this IRF in the obtained distribution from the object 40.

In other words, the system is configured to utilise an obtained time-of-flight distribution from operating in the calibration arrangement to process a time-of-flight distribution obtained from operating in the measurement arrangement. In so doing, the system is configured to identify a time-of-flight distribution attributable to the sample itself (e.g., which has removed the influence of the IRF on the obtained distribution). This sample distribution may be used for analysis of the object 40. For example, the system may be operable to determine an absorption coefficient, a reduced scattering coefficient, a mean time-of-flight, and/or a total intensity based on this sample distribution.

As described above, the present disclosure provides a spectroscopy system configured to provide calibrated analysis of an object 40 to be analysed. For this, the system may be configured to obtain time-of-flight data through the object 40, where that data can be calibrated to take account of an instrument response function for the system itself. While this functionality may be broadly applicable in a large number of technical fields, one particular application of interest is for non-invasive analysis of human or animal bodies, and in particular for analysis of brain tissue.

In view of this, embodiments of the present disclosure may comprise a spectroscopy system configured to provide non-invasive analysis of a human or animal body. The spectroscopy system may comprise a mount for mounting to a surface of a human or animal body. In particular, the spectroscopy system may comprise a head mount configured to mount to the head of a human or animal head, such as the head of a human, e.g., a human adult. The head mount may be configured to couple to the head to enable the system to analyse brain tissue within the head. For example, the head mount may be configured to couple to at least a portion of the surface of the head for directing light towards the subject's scalp and into their brain tissue. The coupling element 130 of the probe 100 may be configured to couple to skin, such as to the scalp or other skin on the head. The adhesive layer 131/coupling surface 135 may be configured to adhere to the skin. The flexible layer 133 may be configured to conform to shape of the head. For example, the head mount may comprise a plurality of such probes 100, e.g., so that multiple light delivery channels 10 and light receiving channels 20 may be directed towards the wearer's brain tissue. In other words, the probe 100 (e.g., the coupling element 130) may be configured to couple to either the reflective element 30 or the head of a user to be analysed.

The system may be configured to determine an intracranial pressure measurement for the wearer's brain. For example, the system may be configured to follow one of the approaches set out in GB2619063 to determine ICP. Additionally, this determination may be made based on using obtained calibration data (e.g., an obtained IRF) from operating the system in the calibration arrangement.

Although not shown in the figures, the system may comprise a controller configured to control operation of the system to implement any of the methods disclosed herein. For example, the controller may be configured to determine an indication of the IRF based on obtained data from received light signals while operating in the calibration arrangement. Likewise, the controller may be configured to utilise obtained calibration data (e.g., the IRF) for processing obtained data from received light signals while operating in the measurement arrangement. For example, the controller may be configured to analyse the object 40 (in the measurement arrangement) taking into account the obtained calibration data (e.g., by deconvolving the IRF from obtained measurement data for the object 40).

It is to be appreciated in the context of the present disclosure that the examples described herein are intended to described aspects of the technology, but these should not be considered limiting. For example, the system is configured to operate in one of two arrangements: (i) a calibration arrangement in which light is reflected from a known reflector, and (ii) a measurement arrangement in which light is reflected from an object to be analysed. In one or both of these arrangements, the system may provide diffusion of light to facilitate the optical path from light delivery channel 10 to light receiving channel 20 (via reflective element 30/object 40). In the examples described above, this may be implemented using one or more layers of optically diffusive material, e.g. layers 32, 132. However, this need not be considered limiting. Instead, the optical diffusion may be implemented using a diffuse reflector, e.g. the reflective element 30 may comprise a diffuse reflector. In the calibration arrangement, this diffusion of light may then be provided using the reflector itself (and not necessarily any other diffuser layer(s)). In such examples, the diffuser layers 32, 132 may still be included, or they may not. For example, alternative layers may be included, such as optically transparent or optically guiding layers.

For example, in the examples shown in the figures, there is one probe 100 which carries two light channels. However, this arrangement should not be considered limiting. For example, one probe 100 may have more light channels (e.g., two or more light delivery channels 10 and/or two or more light receiving channels 20). Alternatively, one probe 100 may only carry one channel (e.g., one of the light delivery channel 10 or the light receiving channel 20). In which case, said probe 100 may be located adjacent to another probe 100 carrying another channel. The two probes 100 may be couplable to a reflective element 30 thereby to enable light to travel from a light delivery channel 10 to a light receiving channel 20 (e.g., for operating in the calibration arrangement).

In examples described herein and as shown in the Figs., the reflective element 30 may be coupled directly to a probe 100 when in the calibration arrangement. Light from the light delivery channel 10 may then reflect from element 30 to be received in light receiving channel 20. When in the measurement arrangement, element 30 is removed and light from channel 10 may pass towards the object 40. In these examples, the reflective element 30 couples to the probe 100 itself, e.g. to the coupling element 130. However, it will be appreciated that embodiments of the present disclosure may also relate to other structural arrangements which provide both the calibration and measurement arrangements. For example, the system may include a separate component which is configured to receive the probe 100. The reflective element 30 may couple to this separate component, e.g. instead of coupling directly to the probe 100. Likewise, this component may be configured to receive the probe 100 and to couple the probe to the object 40. For example, the reflective element 30 may be removed from said component so as to transition the system from the calibration arrangement to the measurement arrangement.

In examples described above and shown in the Figs., there are a number of different configurations for the coupling element 130 and how the light channels 10, 20 are connected within the probe 100 and to the coupling element 130. However, it is to be appreciated that each of these different arrangements should not be considered limiting. For instance, the particular arrangement of the layers of the coupling element 130 and/or how the channels 10, 20 are connected may depend on an intended usage of the probe 100. In some cases, the probe 100 may be a single use device. For instance, in medical scenarios, components may be discarded after they have come into contact with a patient (e.g., human or animal body). The probe 10 may be arranged to permit multiple uses of the light channels 10, 20. In which case, the probe 10 and in particular the coupling element 130 may be configured to provide at least one intervening surface which will be located between the channel ends 110, 120 and the surface of the object to be analysed 40 (e.g., a patient's skin). The intervening surface will be at least partially optically transparent. For example, the intervening surface may comprise a diffuser layer (e.g., the second diffuser layer 132). The intervening surface may be removable, so that it may be removed after use of the probe 10 in a measurement arrangement and replaced with another such intervening surface prior to subsequent use of the probe 10 with another object 40. The channel ends 110, 120 may directly contact the intervening surface (e.g., to minimise the optical path length for light once out of the optical channels) or there may be additional intervening layers or free space. Where the probe 10 itself is designed to be a single-use component, the coupling element 130 may not include any intervening layers between the channel ends 110, 120 and the object to be analysed 40. For example, the channel ends 110, 120 may directly contact the surface of the object 40 (e.g., the patient's skin). The channels 10, 20 may then be discarded after use.

In the layered structure shown in Inset A of Fig. 2a, two diffuser layers are shown (32 and 132). However, only one may be provided. For example, the first diffuser layer 32 may be configured to provide sufficient diffusion for operation in the calibration arrangement. In which case, the channel ends 110, 120 may directly contact the first diffuser layer 32 of the reflective element 30. Then, when operating in the measurement arrangement, no diffusion may be provided, e.g., with the ends 110, 120 directly contacting the object to be analysed (e.g., and the probe 10 may be single use). In examples where the second diffuser layer 132 is included, the first diffuser layer 32 may or may not also be included. Without the first diffuser layer 32, the second diffuser layer 132 may be configured to provide sufficient diffusion for operation in the calibration arrangement. When the first diffuser layer 32 is included, the second diffuser layer 132 may be more optically transparent (e.g., a thinner material) than the first diffuser layer 32. For example, the second diffuser layer 132 may be included to provide an intervening layer between the channel ends 110, 120 and object 40. Also, in Fig. 2a, an adhesive layer 131 is shown although this may be omitted. For example, in the calibration arrangement, the probe 10 may be held against (but not adhered to) the reflective element 30.

In examples described herein, the coupling surface 135 is described as comprising an adhesive (e.g., adhesive layer 131). However, other coupling mechanisms could also be employed. For example, a mechanical coupling may be included, such as for slidably receiving the reflective element 30. Also, it will be appreciated that the coupling surface 135 may comprise the adhesive layer 131 (e.g., it may be the adhesive layer 131). The coupling surface 135 may be the surface of the coupling element 130/probe which contacts the surface of the body to which that probe is to be attached (e.g., the reflective element 30 or the object 40). Similarly, the coupling element 130 and reflective element 30 have each been shown as multi-layered structures. It will be appreciated that this need not be the case, as they could be provided by single layer structures. Alternatively, one or both of these structures may comprise additional layers to those shown in the figures. Likewise, material within the layers could be distributed across a larger or smaller area than shown in the figures. As will be appreciated, these structures are designed to permit an optical path from light source to light detector (through the relevant optical channels), and the coupling element 130 is designed to accommodate such optical paths. Additionally, in the examples described above, there are two diffuser layers (one in the coupling element 130 and one in the reflective element 30), but it will be appreciated that this need not be the case. There could be more or fewer (e.g., 1 or 0) diffuser layers. For example, the reflective element 30 may be configured to provide a specular reflection path without the need for a diffuser layer. The reflective element 30 may not have a diffuser layer and the diffuser layer of the coupling element 130 may provide the relevant diffusion for the calibration arrangement, or vice-versa.

Also, in examples described herein, the calibration arrangement is typically described as being performed first, before performing measurements of the object 40 afterwards.

However, this temporal arrangement is not limiting, as the opposite order could be employed. As will be apparent in the context of the present disclosure, the probe 100 is configured to couple to either surface (of the reflective element 30 or of the object 40). The order in which the two are coupled need not be essential then, as the calibration data could be obtained after all the measurement data for the object 40 has been obtained, with the calibration being performed e.g., digitally, thereafter. Alternatively, the spectroscopy system may be configured to perform the calibration first. For example, the system may be provided with the reflective element 30 pre-installed. For example, the system may comprise the probe 100 already coupled to the reflective element 30, or a user of the system may couple the two together.

It will be appreciated from the discussion above that the examples shown in the figures are merely exemplary, and include features which may be generalised, removed or replaced as described herein and as set out in the claims. With reference to the drawings in general, it will be appreciated that schematic functional block diagrams are used to indicate functionality of systems and apparatus described herein. In addition, processing functionality may also be provided by devices which are supported by an electronic device. It will be appreciated however that the functionality need not be divided in this way, and should not be taken to imply any particular structure of hardware other than that described and claimed below. The function of one or more of the elements shown in the drawings may be further subdivided, and/or distributed throughout the apparatus of the disclosure. In some examples the function of one or more elements shown in the drawings may be integrated into a single functional unit.

As will be appreciated by the skilled reader in the context of the present disclosure, each of the examples described herein may be implemented in a variety of different ways. Any feature of any aspects of the disclosure may be combined with any of the other aspects of the disclosure. For example, method aspects may be combined with apparatus aspects, and features described with reference to the operation of particular elements of apparatus may be provided in methods which do not use those particular types of apparatus. In addition, each of the features of each of the examples is intended to be separable from the features which it is described in combination with, unless it is expressly stated that some other feature is essential to its operation. Each of these separable features may of course be combined with any of the other features of the examples in which it is described, or with any of the other features or combination of features of any of the other examples described herein. Furthermore, equivalents and modifications not described above may also be employed without departing from the invention.

Certain features of the methods described herein may be implemented in hardware, and one or more functions of the apparatus may be implemented in method steps. It will also be appreciated in the context of the present disclosure that the methods described herein need not be performed in the order in which they are described, nor necessarily in the order in which they are depicted in the drawings. Accordingly, aspects of the disclosure which are described with reference to products or apparatus are also intended to be implemented as methods and vice versa. The methods described herein may be implemented in computer programs, or in hardware or in any combination thereof. Computer programs include software, middleware, firmware, and any combination thereof. Such programs may be provided as signals or network messages and may be recorded on computer readable media such as tangible computer readable media which may store the computer programs in non-transitory form. Hardware includes computers, handheld devices, programmable processors, general purpose processors, application specific integrated circuits (ASICs), field programmable gate arrays (FPGAs), and arrays of logic gates.

Other examples and variations of the disclosure will be apparent to the skilled addressee in the context of the present disclosure.

## Claims

1. A method of using a spectroscopy system comprising: (i) a light delivery channel, (ii) a light receiving channel, and (iii) a reflective element, the method comprising:
operating in a calibration arrangement in which light is delivered from the light delivery channel, reflected from the reflective element, and received in the light receiving channel; and
operating in a measurement arrangement in which light is delivered from the light delivery channel, travels through an object to be analysed, and is received in the light receiving channel.

2. The method of claim 1, wherein light delivered from the light delivery channel is diffused prior to reflection from the reflective element and/or by the reflective element in the calibration arrangement, optionally wherein the light is diffused using at least one of: (i) a diffuser layer positioned between the light delivery channel and the reflective element, and (ii) the reflective element.

3. The method of claim 1 or 2, wherein light delivered from the light delivery channel is diffused and/or guided prior to travelling through the object in the measurement arrangement, optionally wherein the light is diffused and/or guided using a material layer positioned between the light delivery channel and the object.

4. The method of any preceding claim, wherein the method comprises transitioning from operating in the calibration arrangement to operating in the measurement arrangement, and wherein said transitioning comprises removing the reflective element.

5. The method of claim 4, wherein removing the reflective element comprises detaching the reflective element from a component which carries the light delivery channel.

6. The method of any preceding claim, further comprising determining an indication of an instrument response function for the spectroscopy system based on light received in the light receiving channel when operating in the calibration arrangement, optionally wherein analysing the object in the measurement arrangement is based on both: (i) light signals received from the object in the light receiving channel, and (ii) the determined instrument response function.

7. A spectroscopy system comprising:
a light delivery channel;
a light receiving channel; and
a reflective element;
wherein the spectroscopy system is configurable in:
a calibration arrangement in which light delivered from the light delivery channel is reflected from the reflective element and received in the light receiving channel; and
a measurement arrangement in which light delivered from the light delivery channel travels through an object to be analysed and is received in the light receiving channel.

8. The system of claim 7, further comprising a controller configured to determine an indication of an instrument response function for the system based on light received in the light receiving channel when in the calibration arrangement, optionally wherein the controller is configured to analyse the object based on both: (i) light signals received from the object in the light receiving channel, and (ii) the determined instrument response function.

9. The system of claim 7 or 8, further comprising a probe arranged to carry at least a portion of one or both of the light delivery channel and the light receiving channel.

10. The system of claim 9, wherein the probe comprises at least one intervening layer arranged between the light delivery channel and the reflective element and/or the object.

11. The system of claim 9 or 10, wherein the reflective element is removably couplable to the probe and/or a component arranged to couple the probe to a surface of the object.

12. The system of any of claims 7 to 11, wherein transitioning from the calibration arrangement to the measurement arrangement comprises removing the reflective element to permit light to travel from the light delivery channel towards the object, optionally wherein the reflective element comprises a tab to facilitate removal of the reflective element.

13. The system of any of claims 7 to 12, wherein the system comprises a coupling surface configured to couple the light delivery channel and/or the light receiving channel to: (i) the reflective element in the calibration arrangement, and (ii) the object to be analysed in the measurement arrangement, optionally wherein the coupling surface comprises an adhesive.

14. The system of any of claims 7 to 13, wherein at least one of: (i) the system comprises a diffuser layer arranged to diffuse light output from the light delivery channel, and (ii) the reflective element comprises a diffusive reflector, optionally wherein the diffuser layer comprises a mylar film.

15. The system of any of claims 7 to 14, wherein the reflective element comprises a reflective film, and/or wherein the spectroscopy system is a time-resolved spectroscopy system, optionally an iNIRS system.
